# EUROPEAN PATENT APPLICATION

(11) **EP 4 570 233 A1**
(43) Date of publication of application: **18.06.2025**
(21) Application number: 23216976.3
(22) Date of filing: 15.12.2023
(51) Int. Cl.: A61K 8/19, A61K 8/44, A61Q 11/00

(54) **DENTIFRICE COMPOSITION BASED ON BICARBONATE**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The invention related to a dentifrice composition comprising at least 50 % by weight of sodium bicarbonate and between about 0.2 % to about 5 % by weight of a surfactant system comprising of at least about 0.1 % by weight of sodium cocoyl glycinate in combination with at least about 0.1 % by weight of a betaine.

## Description

### Field of invention

The present invention relates to an aqueous sodium bicarbonate dentifrice composition comprising a novel surfactant system. In particular the invention relates to a dentifrice composition comprising sodium bicarbonate with a combination of surfactants comprising sodium cocoyl glycinate and a betaine. Such a dentifrice composition demonstrates stability, good foam volume and pleasant organoleptic properties.

### Background to the invention

The use of sodium bicarbonate in dentifrices products is well known. One example is the commercial product Paradontax/Corsodyl which contains 67 % by weight of sodium bicarbonate.

Any dentifrice, in particular a sodium bicarbonate dentifrice must be acceptable from a consumer standpoint and demonstrate, for example, acceptable taste, consistency and adequate foaming on brushing of teeth. Any such composition must also be stable and not suffer from syneresis, which is the contraction of a gel accompanied by the separating out of liquid.

WO2019/057809 disclosed the difficulties in working with high bicarbonate toothpastes and is herein incorporated by reference.

There is a continual desire to improve the foaming performance of the bicarbonate pastes.

### Summary of the invention

In its broadest aspect the invention discloses a dentifrice composition comprising at least 50 % by weight of sodium bicarbonate and between 0.2 % to 5 % by weight of a surfactant system comprising of at least 0.1 % by weight of sodium cocoyl glycinate in combination with at least 0.1 % by weight of a betaine.

In a further embodiment the sodium cocoyl glycinate comprises between 0.2 % to 0.8 % by weight of the dentifrice composition.

In a further embodiment the betaine is cocoamidopropyl betaine.

In a further embodiment the betaine is present in an amount between about 0.2 % and about 0.8 % by weight.

In a further embodiment the weight ratio of surfactants (sodium cocoyl glycinate: betaine) is between 9:10 and 10:9.

In a further embodiment the sodium bicarbonate is present in an amount from about 55 % to about 75 % by weight of the composition.

In a further embodiment the composition comprises between about 10 % to about 20 % by weight of water.

In a further embodiment the composition further comprises a humectant between about 5 % and about 10% by weight.

In a further embodiment the dentifrice further comprises a fluoride source, a desensitizing agent, an abrasive, an anti-plaque agent, an anti-calculus agent, a whitening agent, an oral malodour agent, an anti-inflammatory agent, an antimicrobial agent, an anti-oxidant, an anti-fungal agent, a wound healing agent or a mixture of at least two thereof.

In a further embodiment the dentifrice composition further comprises a sweetener, a colourant, a flavouring, a pH modifier, a thickening gun or a mixture of at least two thereof.

In a further embodiment the dentifrice composition comprises:
Sodium bicarbonate between about 60 and about 70 % by weight
Water between about 12 % and about 15 % by weight
Humectant between about 5 % and about 10 % by weight
Sodium cocoyl glycinate between about 0.4 % and about 0.75 % by weight; and
Cocamidopropyl betaine between about 0.4 % and about 0.75 % by weight.

In a further embodiment the dentifrice composition further comprises alumina as an abrasive.

In a further embodiment the dentifrice composition further comprises an abrasive silica.

In a further embodiment the dentifrice composition further comprises a spherical silica.

In a further embodiment the dentifrice composition comprises:
Sodium bicarbonate between about 62 % and about 68 % by weight;
Water between about 12 % and about 15 % by weight;
Glycerol between about 5 % and about 10 % by weight;
Sodium cocoyl glycinate between about 0.50 % and about 0.65 % by weight;
Cocamidopropyl betaine between about 0.50 % and about 0.65 % by weight; and
A source of fluoride ions.

### Detailed description of the invention.

The dentifrice compositions of the present invention comprise sodium bicarbonate as the main component. Suitably the sodium bicarbonate is present in an amount from about 55% to about 90% by weight preferably from about 60 % to about 70 % by weight. e.g. from about 62% to about 68% w/w.

The high bicarbonate based dentifrice composition according to the invention comprises a surfactant system. The surfactant system consists of sodium cocyl glycinate and a betaine present in a total amount of 0.2 % to 5 % by weight of the dentifrice, preferably in a total amount of 0.6 % to 1.60 % by weight.

Preferably the betaine is Cocamidopropyl Betaine. A suitable commercial source of these the Tego^{®} range of surfactants from Evonik.

Sodium cocoyl glycinate can be sourced commercially from a number of suppliers including Amilite ^{®} range from Ajinomoto.

The dentifrice compositions of the present invention may comprise may further ingredients suitable for use in dentifrice compositions.

The dentifrice composition accord to the present invention may comprise a humectant.

Non-limiting examples of suitable humectants for use in compositions of the invention include glycerin, xylitol, sorbitol, propylene glycol or polyethylene glycol, or mixtures of at least two thereof;

A particularly preferred humectant is glycerin.

The humectant may comprise between about 1% and about 20% by weight of the dentifrice composition, more preferably between about 2.5 % and about 15 % by weight of the dentifrice composition and most preferably between about 5 % and about 10% by weight of the dentifrice composition.

The dentifrice compositions of the present invention may comprise water.

The water may comprise between about 1% and about 20% by weight of the dentifrice composition, more preferably between about 5 % and about 17.5 % by weight of the dentifrice composition and most preferably between about 10 % and about 15 % by weight of the dentifrice composition.

The dentifrice composition according to the invention may comprise further surfactants.

The invention is not limited to compositions comprising particular types or amounts of surfactants. The skilled person will be aware of suitable further surfactants that can be used with the compositions of the present invention.

In addition to the above ingredients, compositions of the present invention may comprise a fluoride source, a desensitising agent, an anti-plaque agent, an anti-calculus agent, a whitening agent, an oral malodour agent, an anti-inflammatory agent, e.g. monoammonium glycyrrhizinate (MAG), an antimicrobial e.g. isopropylmethylphenol (IPMP), an anti-oxidant, an anti-fungal agent, a wound healing agent, e.g. hyaluronic acid, or a mixture of at least two thereof. Such agents may be included at levels to provide the desired therapeutic effect.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25ppm to 3500 ppm of fluoride ions, preferably from 100 ppm to 1500 ppm. A typical fluoride source is sodium fluoride, for example the composition may contain 0.1 % to 0.5 % by weight of sodium fluoride, e.g. 0.204 % by weight (equating to 927ppm of fluoride ions), 0.2542 % by weight (equating to 1150 ppm of fluoride ions) or 0.315 % by weight (equating to 1426 ppm of fluoride ions).

Such fluoride ions help promote the remineralisation of teeth and can increase the acid resistance of dental hard tissues for combating caries, dental erosion (i.e. acid wear) and/or tooth wear.

Sodium bicarbonate will provide abrasive properties to the composition however an additional abrasive may also be present, e.g. a silica that has high cleaning properties. Examples of high cleaning silica abrasives include those marketed as Zeodent 124, Tixosil 63, Sorbosil AC39, Sorbosil AC43 and Sorbosil AC35 and may be present in suitable amounts for example up to 20 %, such as from about 5 % to about 10 %, preferably from about 1.5 % to about 7 %, e.g. 2 % by weight of the total composition.

Compositions of the present invention will contain additional formulating agents such as flavouring agents, sweetening agents, opacifying or colouring agents and preservatives, selected from those conventionally used in an oral hygiene composition art for such purposes.

In addition to the above ingredients, compositions of the present invention may comprise a fluoride source, a desensitising agent, an anti-plaque agent, an anti-calculus agent, a whitening agent, an oral malodour agent, an anti-inflammatory agent, e.g. monoammonium glycyrrhizinate (MAG), an antimicrobial e.g. isopropylmethylphenol (IPMP), an anti-oxidant, an anti-fungal agent, a wound healing agent, e.g. hyaluronic acid, or a mixture of at least two thereof. Such agents may be included at levels to provide the desired therapeutic effect.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 to 3500 ppm of fluoride ions, preferably from 100 ppm to 1500 ppm. A typical fluoride source is sodium fluoride, for example the composition may contain 0.1 % to 0.5 % by weight of sodium fluoride, e.g. 0.204 % by weight (equating to 927 ppm of fluoride ions), 0.2542 % by weight (equating to 1150ppm of fluoride ions) or 0.315 % by weight (equating to 1426 ppm of fluoride ions).

Such fluoride ions help promote the remineralisation of teeth and can increase the acid resistance of dental hard tissues for combating caries, dental erosion (i.e. acid wear) and/or tooth wear.

Sodium bicarbonate will provide abrasive properties to the composition however an additional abrasive may also be present, e.g. a silica that has high cleaning properties. Examples of high cleaning silica abrasives include those marketed as Zeodent 124, Tixosil 63, Sorbosil AC39, Sorbosil AC43 and Sorbosil AC35 and may be present in suitable amounts for example up to 20 %, such as from 5 % to 10 %, preferably from 1.5 % to 7 %, e.g. 2 % by weight of the total composition.

Suitable humectants for use in compositions of the invention include glycerin, xylitol, sorbitol, propylene glycol or polyethylene glycol, or mixtures of at least two thereof; which humectant may be present in the range from 1.0 % to 20 %, for example from 3 % to 15 % or from 5 % to 10 % by weight of the total composition.

### Tests

The following base formulation was prepared:

**Table 1**

| **Raw Material** | **% w/w** |
|---|---|
| Water | 18.5 |
| Sodium Bicarbonate | 67.3 |
| Glycerol | 7.5 |
| Xanthan Gum | 0.7 |
| Sodium Fluoride | 0.3 |
| Abrasive | 1.2 |
| Surfactant (base) | 1.2 |
| Minor ingredients : Flavour/colour/sweetener etc | 3.3 |
| Total | 100 |

The test formulations we all surfactant variations on this base formulation. (Where more or less surfactant was included, the level of water was adjusted accordingly)

A large number of surfactants were trialled in stability and foaming tests.

In screening surfactants it was quickly found that sodium cocoyl glycinate was the best performing single surfactant at the 1.5 % by weight level.

To test this for foaming performance the following samples were made up.

**Table 2**

| **Surfactant systems tested in for foam height** |
|---|
| 1.20 % cocamidopropyl betaine (commercial control) |
| 0.75 % sodium cocoyl glycinate |
| 0.90 % sodium cocoyl glycinate |
| 1.50 % sodium cocoyl glycinate |
| 0.94 % cocamidopropyl betaine and 0.9 % sodium cocoyl glycinate |
| 1.175 % cocamidopropyl betaine and 0.75 % sodium cocoyl glycinate |
| 1.67 % cocamidopropyl betaine and 0.9 % sodium cocoyl glycinate |

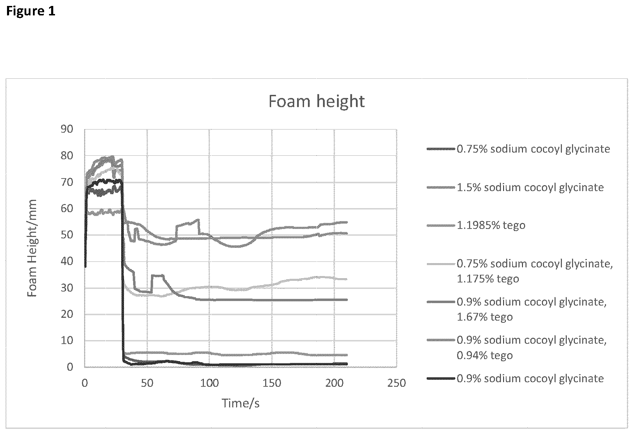

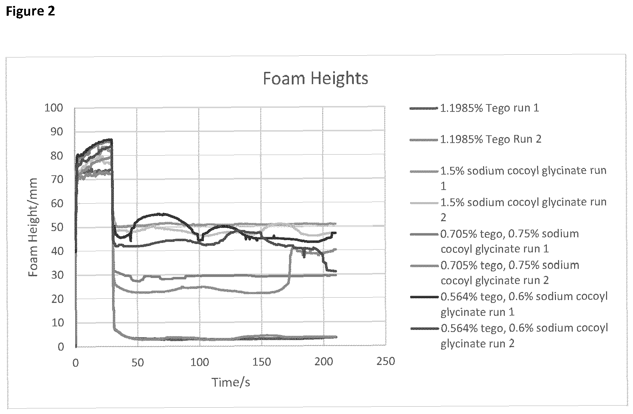

The formulas above were tested in a foam analyser The results are shown in Figure 1.

The two best surfactant systems use were 1.5 % sodium cocoyl glycinate and the composition comprising the the near 1:1 ratio of cocamidopropyl betaine (0.94 %) with sodium cocoyl glycinate (0.9%).

Interestingly, increasing the ratio of cocamidopropyl betaine to sodium cocoyl glycinate lead to worse foaming performance.

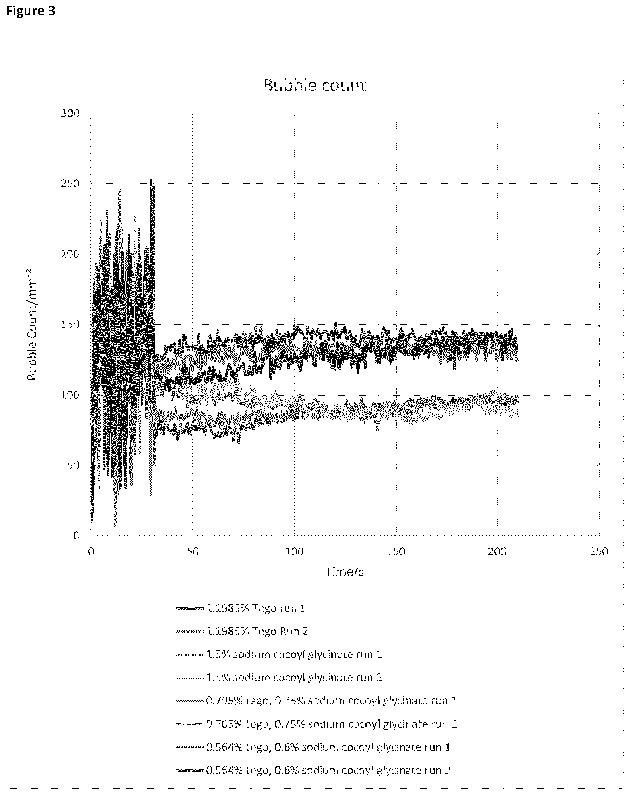

This result to a desire to see whether the 1:1 combination could be further optimised for using less surfactant and the following two formulas were prepared for testing against the single surfactant control. The foam volume test results are shown in Figure 3.

**Table 3**

| **Optimised level testing compositions:** |
|---|
| 0.56 % cocamidopropyl betaine and 0.60 % sodium cocoyl glycinate |
| 0.70 % cocamidopropyl betaine and 0.75 % sodium cocoyl glycinate |
| 1.50 % sodium cocoyl glycinate (control) |
| 1.20 % cocamidopropyl betaine (control) |

A near 1:1 ratio of less than 1.16 % by weight sodium cocoly glycinate and tego betaine was found to be as effective as 1.5 % sodium cocoyl glycinate alone in foaming volume.

Interestingly the surfactant levels seem highly sensitive to change.

An increase in the levels of both surfactants to 0.7 % cocamidopropyl betaine and sodium cocoyl glycinate 0.75 % respectively gave rise to a worse performance than the dentifrice composition comprising either 0.56 % cocamidopropyl betaine 0.60 % sodium cocoyl glycinate and the dentifrice composition comprising 0.94 % cocamidopropyl betaine and 0.90 % sodium cocoyl glycinate.

This combination also provided the foam with the highest number of bubbles. (see Figure 3)

It was found that the number of bubbles and bubble size showed a correlation - the higher the number of bubbles there was the smaller the bubbles were. This would represent a creamier and denser foam.

### Foam height test method

### Bubble count test method

The results were all generated on the Kruss dynamic foam analyser with the below parameters.
Stirring Speed - 5000rpm
Foaming Time - 30 seconds
Measurement Time - 3 Minutes

## Claims

1. A dentifrice composition comprising at least 50 % by weight of sodium bicarbonate and between about 0.2 % to about 5 % by weight of a surfactant system comprising of at least about 0.1 % by weight of sodium cocoyl glycinate in combination with at least about 0.1 % by weight of a betaine.

2. The composition of claim 1 wherein the sodium cocoyl glycinate comprises between about 0.2 % to about 0.8 % by weight.

3. The composition according to either claim 1 or claim 2 wherein the betaine is cocamidopropyl betaine.

4. The composition according to any of the previous claims wherein the betaine is present in an amount at least between about 0.2 % and about 0.8 % by weight.

5. The composition according to any of the prevision claims wherein the weight ratio of surfactants (sodium cocoyl glycinate: betaine) is between 9:10 and 10:9.

6. The composition according to any one of claims 1 to 4 wherein the sodium bicarbonate is present in an amount from about 55 % to about 75 % by weight of the composition.

7. The composition according to any of the previous claims wherein the composition comprises between about 10 % to about 20 % by weight of water.

8. The composition according to any one of the previous claims wherein the composition further comprises a humectant between about 5 % and about 10 % by weight.

9. The composition according to any one of claims 1 to 7 additionally comprising a fluoride source, a desensitizing agent, an abrasive, an anti-plaque agent, an anti-calculus agent, a whitening agent, an oral malodour agent, an anti-inflammatory agent, an antimicrobial agent, an anti-oxidant, an anti-fungal agent, a wound healing agent or a mixture of at least two thereof.

10. The composition according to any of the previous claims comprising a sweetener, a colourant, a flavouring, a pH modifier, a thickening gun or a mixture of at least two thereof.

11. The dentifrice composition according to any of the previous claims wherein the composition comprises:
Sodium bicarbonate between about 60 % and about 70 % by weight;
Water between about 12 % and about 15 % by weight;
Humectant between about 5 % and about 10 % by weight;
Sodium cocoyl glycinate between about 0.4 % and about 0.75 % by weight; and
Cocamidopropyl betaine between about 0.4 % and about 0.75 % by weight.

12. The dentifrice composition according to any of the previous claims wherein the composition comprises:
Sodium bicarbonate between about 62 % and about 68 % by weight;
Water between about 12 % and about 15 % by weight;
Glycerol between about 5 % and about 10 % by weight;
Sodium cocoyl glycinate between about 0.50 % and about 0.65 % by weight;
Cocamidopropyl betaine between about 0.50 % and about 0.65 % by weight; and
A source of fluoride ions.

13. The dentifrice composition according to any of the previous claims wherein the composition further comprises an abrasive.
